Europäisches Patentamt

European Patent Office

Office européen des brevets

(11) Publication number: **0 248 637**
A2

# EUROPEAN PATENT APPLICATION

(21) Application number: **87304870.6**

(22) Date of filing: **02.06.87**

(51) Int. Cl.⁴: **C 12 N 15/00**
**C 12 P 21/00**

(30) Priority: **03.06.86 GB 8613388**

(43) Date of publication of application:
**09.12.87 Bulletin 87/50**

(84) Designated Contracting States:
**AT BE CH DE ES FR GB GR IT LI LU NL SE**

(71) Applicant: **Delta Biotechnology Limited**
**137 High Street**
**Burton on Trent DE14 1JZ (GB)**

(72) Inventor: **Hinchliffe, Edward**
**16, Lambley Lane**
**Burton Joyce Nottingham (GB)**

(74) Representative: **Collier, Jeremy Austin Grey et al**
**J.A.Kemp & Co. 14, South Square Gray's Inn**
**London WC1R 5EU (GB)**

(54) Induction of galactose regulated gene expression in yeast.

(57) A process for the production of a protein or peptide which is heterologous to yeast which comprises inducing the expression of the protein or peptide in a yeast, which has been genetically modified to be capable of expressing the heterologous protein or peptide upon induction by galactose, the induction being carried out in the presence of a galactose-containing medium which contains a significant amount of glucose, and obtaining the said heterologous protein or peptide therefrom.

EP 0 248 637 A2

**Description**

INDUCTION OF GALACTOSE GENE EXPRESSION IN YEAST

This invention relates to the induction of galactose regulated gene expression in yeast.

The yeasts are a group of lower eukaryotic micro-organisms showing biological and biochemical diversity. In common usage the term "yeast" is used to describe strains of Saccharomyces cerevisiae that have commercial value in baking, brewing and distilling. Related yeasts are used in wine making and sake brewing, as well as in the production of fuel alcohol from sucrose or hydrolysed starch, and in the treatment of effluents.

All the yeasts used for brewing, baking and distilling may be taxonomically classified as Saccharomyces cerevisiae. Included within this classification are the top fermenting ale yeasts (S. cerevisiae) and the bottom-fermenting lager yeasts (S. uvarum or S.Carlsbergensis).

In a strict sense the term "brewers yeast" differentiates yeast used in brewing from all other yeasts in that it is a yeast strain which is used to make beer, i.e. a strain of yeast used currently in a beer manufacturing process. Such yeast must be able to produce a palatable acceptable beer by their fermentative action upon hopped or unhopped malt extract (brewers wort). The primary products of this fermentation are ethanol and carbon dioxide, which are essential constituents of beer. However, not all yeasts belonging to the species S. cerevisiae are capable of fulfilling these requirements. Indeed, the critical factor in this respect is believed to be the ability of the yeast strain to form in subtly balanced proportions, quantitatively minor metabolic products such as esters, acids, higher alcohols and ketones. A yeast may be unsuitable for brewing because one or more of these minor metabolic products is produced in excessive amounts, either in absolute terms or relative to one another. (Rainbow, C.A., 1970, In "The Yeasts". eds, Rose, A.H. & Harrison J.S. Vol. 3, p. 147).

In a general sense brewers yeast is differentiated from other yeasts by the properties which it possesses. Most strains of industrial yeast, unlike laboratory yeast, are incapable of undergoing mating; they are said to be homothallic. Industrial yeasts are usually aneuploid or polyploid, and there is therefore a reduced incidence at which gene mutations are phenotypically detected. Most polyploid strains do not sporulate or they produce spores of very low viability, thus frustrating any meaningful genetic analysis. These factors together tend to confer a measure of phenotypic stability on industrial yeasts which may contribute to their selection for industrial application. Similarly gene dosage which is associated with high ploidy may contribute to the general fitness of such strains for fermentation as compared to haploids and diploids, which generally ferment poorly.

In addition, brewers yeasts have certain technological behaviour which equips them well for interacting with their normal environment, i.e. brewers' hopped wort, for example the ability to ferment at the top of the fermentation vessel (ale yeast) or the bottom of the vessel (lager yeast).

In European Patent Application No. 84308981.4 in the name of Bass PLC, published under No. 147198 there is described and claimed a process for the production of ethanol and a protein or peptide which is heterologous to yeast which comprises fermenting an aqueous carbohydrate-containing medium with a yeast strain which has been genetically modified to be capable of expressing a heterologous protein or peptide, recovering the ethanol so formed, and obtaining the said heterologous protein, or peptide from the fermentation products.

The invention of that Application is based on the discovery that it is possible to use, in an industrial fermentation involving the production of alcohol, genetically modified yeast capable of expressing a heterologous protein or peptide. Surprisingly, it was found that the use of such yeasts is compatible with industrial or brewery fermentation conditions. This means that the excess yeast obtained in the fermentation provided a source of the heterologous protein or peptide and thus has much enhanced industrial value. Further, since the alcohol product remains the principal objective of the fermentation, and the conventional equipment can largely be used with little alteration, the additional cost of producing the higher value yeast product is small, so that the new process may provide an economically viable route to heterologous proteins or peptides which, although valuable, do not command a premium price.

When the process of the aforesaid Application is operated to produce an aqueous potable liquid such as beer, at the end of the fermentation, the fermented liquid is separated from the yeast (and normally any other solid material present in the fermented medium). In these circumstances, it is clearly desirable, and indeed normally essential, that the fermented liquid shall not contain the heterologous protein or peptide, since it is normally unacceptable for the heterologous protein or peptide to be present in a liquid which is to be drunk.

In the said Application it is stated that, in such circumstances, the heterologous protein or peptide may be obtained from the yeast cells.

In European Patent Application No.86303039.1 (Isolation of Fermentation Products) in the name of Delta Biotechnology Ltd., published under No.201239 there is described and claimed a process for the production of ethanol and a protein or peptide which is heterologous to yeast, which comprises fermenting an aqueous carbohydrate-containing medium with a yeast strain, which has been genetically modified to be capable of expressing heterologous protein or peptide, recovering the ethanol so formed, and obtaining the said heterologous protein or peptide from the waste yeast, by a process of post-fermentation induction. Normally the expression of the heterologous protein or peptide is induced after the yeast has been separated from the fermented liquid, and subsequently the heterologous protein or peptide is separated from the yeast. Operation in this manner avoids the risk that the fermented liquid itself may become contaminated with the heterologous protein or peptide. Furthermore it avoids any risk of the fermentation performance of the yeast being comprised by the presence of the heterologous protein or peptide and/or the synthesis of the heterologous protein or peptide.

Various genetic systems have been identified as having potential for regulating the expression of heterologous genes in brewing yeast in accordance with the method described in the said European Patent Application No.86303039.I. One genetic system which is particularly useful for application in this process uses galactose to effect the expression of a galactose regulated gene. This is achieved by fusing a heterologous gene, for example the Human Serum Albumin gene or the LacZ gene of E.coli, to a yeast gene promoter which mediates the galactose induction process. In the aforementioned European patent Application No.86303039.I a DNA sequence, the GALI0 upstream activation sequence, is described which was fused upstream (5') of the CYCI messenger RNA start site to form a hybrid yeast promoter, GALI0/CYCI (Guarente et al, 1982, Proceedings of the National Academy of Science, USA; 79, 74I0-74I4). This hybrid promoter is regulated in a manner analogous to the GALI0 gene of yeast, that is, it is induced by galactose and repressed by glucose. Thus, when yeasts harbouring, for example, plasmid pLGSD5, which contains the GALI0/CYCI promoter fused to the LacZ gene of E.coli are grown in a medium containing galactose they produce twenty-fold more β-galactosidase enzyme than when grown on a medium containing both galactose and glucose, thereby implying that glucose is capable of repressing transcription from the GALI0/CYCI promoter (Guarente et al, 1982).

However, although galactose is effective for inducing heterologous protein production in genetically modified brewing yeast by a post-fermentation induction process, the use of such a system has significant economic limitations because of the expense of pure commercial galactose. Cheaper sources of commercial galactose are available but these contain significant quantities of glucose.

For example, commercial sources of galactose derived from whey permeates, a waste product of the dairy industry, or from the enzymic and/or chemical hydrolysis of lactose, contain substantial amounts of glucose. Cheap galactose is also available as a constituent of hydrolysed raffinose, a waste product of the sugar beet refining industry. Whereas analytical and reagent grade galactose normally contain less than 0.0I% and less than I% glucose respectively, milk sugar may contain up to 40 or 45% glucose and the hydrolysis product of lactose may contain 50% glucose by weight relative to the total weight of the sugars present.

Since it has previously been found that the promoter which mediates galactose induction is repressed by glucose in generically modified brewing yeast, it would be expected that galactose induction of gene expression cannot satisfactorily be performed in a medium which contains glucose. It has, however, surprisingly been found that genetically modified brewing yeast can be induced to produce significant quantities of heterologous protein or peptide by a post-fermentation induction process in the presence of a galactose medium which contains substantial amounts of glucose.

Accordingly the present invention provides a process for the production of a protein or peptide which is heterologous to yeast which comprises inducing the expression of the protein or peptide in a yeast, which has been genetically modified to be capable of expressing the heterologous protein or peptide upon induction by galactose, the induction being carried out with a galactose-containing medium which contains a substantial proportion, normally greater than I% by weight, of glucose relative to the total weight of the galactose and glucose present, and obtaining the said heterologous protein or peptide from the said medium.

Thus the process of the present invention can be carried out in the presence of an amount of glucose which would inhibit expression of the protein or peptide if the yeast were in its growth phase on a medium containing glucose.

The galactose-containing medium normally contains more than I% glucose by weight, for example 5% to 50% glucose by weight, relative to the total weight of galactose and glucose present.

The yeast may conveniently be obtained from the brewing industry operated with yeast which has been genetically modified. Preferably the yeast is obtained as a product of a fermentation process which is carried out in an aqueous carbohydrate-containing medium under such conditions that the yeast multiplies and no expression of the heterologous protein or peptide takes place.

The brewing yeast is preferably a genetically engineered modification of an industrial strain of Saccharromyces cerevisiae or S.carlsbergensis, preferably brewing yeast NCYC 240, BB6, BBI0.I, or BBII.

The fermentation which may produce the yeast used in the present invention is carried out under conditions such that the yeast reproduces in the usual way but little or no heterologous protein or peptide is produced. Then, normally after the yeast has been separated from the fermented liquid, expression of the heterologous protein or peptide is induced, and the protein or peptide so obtained is separated from the yeast.

The induction of heterologous gene expression in yeast which is used for brewing must be compatible with the demands of the beer fermentation if it is to be used in the present invention. In this respect it is necessary to induce gene expression with an effector or condition which is normally not present in brewers' wort and beer. The gene expression system for inducing the production of heterologous proteins and polypeptides in yeast and suitable for use in the present invention utilizes galactose to stimulate gene expression and is particularly useful since brewers' wort does not usually contain sufficient galactose to effect the expression of a galactose regulated promoter. Consequently, such galactose-regulated gene expression systems are not functional in brewing yeast during the course of a beer fermentation.

The yeast strain used in the new process must, of course, be suitable for the type of industrial fermentation contemplated if this is the manner in which the yeast is prepared. This objective may be secured by carrying out the genetic modification on a yeast strain which is known to have the desired characteristics, since it has been found that the desirable characteristics which make a yeast strain suitable for a particular type of industrial fermentation can be maintained during the genetic modification. For example, where the fermentation is one for producing beer, the yeast strain chosen for genetic modification is preferably a known strain of brewers' yeast currently used in such fermentations. As already noted, such industrial strains of brewers yeast have characteristics different from those of "laboratory yeast", including in particular the ability to ferment hopped brewers wort.

Similarly, where the industrial fermentation is one for the production of alcohol which is separated by distillation, it is necessary to use genetically modified yeast obtained from a strain suitable for such fermentation. In such fermentations, the source of carbohydrates may be, for example, grain, potatoes, cassava, sugar cane, sugar beet or a lignocellulosic material and may optionally have been pre-treated, e.g. by chemical, enzymic or physical methods, to convert cellulose and/or starch therein into fermentable sugars.

The genetic modification of yeast may be effected by the application of known techniques. Suitable methods are described in the literature, and particular methods are given in the Examples below.

After the industrial fermentation has been carried out in the usual manner, but using the genetically modified yeast, the yeast is separated and induction of the desired heterologous protein or peptide is initiated by exposing the yeast to the galactose-containing medium. While the galactose may be added to the yeast in a single batch, it is generally preferable to add the galactose-containing medium gradually to the yeast in a fed-batch process.

A wide range of heterologous proteins or peptides may be expressed in the yeast. By way of example mention may be made of enzymes such as beta-lactamase, beta-glucanase, and beta-galactosidase. Other useful heterologous proteins and peptides include materials of human origin and/or useful in therapy, such as human serum albumin and immunoglobulins. Methods are described in the literature for the genetic modification of microorganisms to enable them to express such proteins and peptides.

The following Examples I, II and III illustrate the preparation of materials and the processes used in the present invention. Examples IV and V illustrate the present invention. Unless otherwise stated all the methods and materials used in Examples III, IV and V are as described in Examples I and II. The ten figures of the accompanying drawings show respectively:

I. Construction of plasmid pEHBII

2. Oligonucleotide sequence of the HSA primer

3. DNA sequence of the HSA cDNA gene (see, for example, European Patent Specification published under No. 79739)

4. 5' Non-coding region modification and signal peptide sequence excision

5. Construction of an "authentic" HSA cDNA (MET-HSA)

6. Plasmid pEKII3

7. Construction of plasmid pEHBII-MET-HSA

8. Construction of plasmid pETI3:I MET-HSA

9. Galactose induction of Gene Expression in NCYC 240 (pEHBII) in the absence or presence of glucose.

I0. Plasmid pEKI0I4ALBI

EXAMPLE I

Beta-galactosidase production in Brewing Yeast

Beta-Galactosidase (EC 3.2.I.23) is an enzyme (protein) which hydrolyses the terminal non-reducing beta-D-galactose residues in beta-D-galactosides such as lactose. E.coli beta-galactoside is the product of the Lac Z gene which is located at map position 8 minutes on the E.coli chromosome (Bachmann, B.J., I983, Microbiological Reviews, 47, p. I80). The Lac Z gene forms part of the lac operon, which has played a central role in the elucidation of the genetic control of gene activity in the prokaryote E. coli (Beckwith, J., I972. In "The Operon" p. II, eds. Miller, J.H. and Reznikoff, W.S., Cold Spring Harbor Laboratory, Cold Spring Harbor, New York).

In recent years the structural gene for beta-galactosidase (Lac Z) has been used in DNA manipulations. It has been shown that the amino-terminal end of the beta-galactosidase protein is not essential for enzymatic activity (Muller-Hill, B. and Kania, J., I974, Nature, I49, p.56I). This has facilitated the construction of many gene fusions, in which the 5' - coding segment of the Lac Z gene (corresponding to the N-terminal end of the protein) has been replaced with other DNA sequences. In these gene fusions, the gene transcriptional promoter of Lac Z is replaced by analogous DNA sequences originating from different genes (Casadaban, M.J. et al, I980, Journal of Bacteriology, I43, p. 97I), but proteins obtained retain beta-galactosidase activity.

The Lac Z gene of E.coli was one of the first prokaryotic chromosomal genes to be expressed in S. cerevisiae (Panthier, J.J. et al, I980, Current Genetics, 2, p. I09). In this demonstration the native E.coli Lac Z gene was sub-cloned into a yeast/E. coli shuttle vector (plasmid) and introduced into laboratory strains of yeast which do not possess endogenous beta-galactosidase activity. However, the level of gene expression and thus enzyme production was inefficient using this system. More recently, gene fusions have been constructed in which the transcriptional promoter of the yeast cytochrome CI gene (CYCI) has replaced that of Lac Z. These CYCI - Lac Z fusions have been introduced, by transformation, into laboratory strains of yeast, where they have been shown to produce enzymatically active beta-galactosidase (Guarente, L. and

Ptashne, M. 1981, Proceedings of the National Academy of Sciences, U.S.A., 78, p. 2199). Detailed analysis of the CYCI - Lac Z gene fusions has shown that the levels of beta-galactosidase produced in yeast display the pattern of regulation normally seen for cytochrome c (i.e. a reduction of synthesis in cells grown in glucose). (Guarente, L. and Ptashe, M., 1981). In this way the Lac Z gene has been used to study the regulation of gene expression in both prokaryotes and eukaryotes at the level of DNA transcription.

In the yeast CYCI gene, DNA sequences which regulate DNA transcription by RNA polymerase II have been located in two regions upstream (5') of the coding sequence for the cytochrome c protein. One of these regulatory sequences is situated close to where transcription initiates; the other is upstream of the initiation region and contains an activation site which enhances gene expression (UASc) (Guarente, L. and Ptashne, M. 1981; Faye, G. et al, 1981, Processings of the National Academy of Sciences, U.S.A., 78 p. 2258). Guarente and co-workers (1982, Proceedings of the National Academy of Sciences, 79, p. 7410) have shown that other genes possess upstream activation sites (UAS), in particular the GALI0 gene of S. cerevisiae. These workers constructed a gene fusion in which the UASc of CYCI was replaced by the UAS of the GALI0 gene. Furthermore, the gene fusion which retains the promoter (transcription initiation region) of CYCI is fused to the Lac. Z gene of E. coli, such that an enzymatically active beta-galactosidase is produced in yeast transformed with plasmid DNA carrying the hybrid gene (plasmid pLGSD5).

The regulation of genes involved in galactose metabolism in yeast is well understood. The transcriptional expression of three genes (GALI, GALI0 and GAL7 encoding enzymes required for the metabolism of galactose is dependent upon a transcriptional activator encoded by the gene GAL4 (Oshima, Y. 1982, in "The Molecular Biology of the Yeast Saccharomyces: Metabolism and Gene Expression" eds. Broach, J.R., et al, p.159, Cold Spring Harbor Laboratory, Cold Spring Harbor, New York). The ability of the GAL4 activator protein to promote transcription of these genes is reversibly inactivated by the product of the GAL80 gene, (GAL80 repressor protein). Galactose is thought to bind to the GAL80 repressor protein thereby freeing the GAL4 activator protein to promote transcription of the galactose- specific genes. The GALI0-CYCI hybrid promoter is subject to induction by galactose in the manner described above (Guarente, L. et al, 1982, Proceedings of the National Academy of Sciences, U.S.A., 79, p. 7410). Thus beta-galactosidase production is induced by galactose. The production of E. coli beta-galactosidase in brewing yeast

In order to introduce the lac-Z gene of E. coli, expressed from the GALI0-CYCI hybrid promoter into brewing yeast strain NCYC240, it was necessary to sub-clone the CUP-I gene present on plasmid pETI3:I.The accompanying Figure I outlines a scheme whereby the XbaI DNA fragment of approximately 2.85 kilo base pairs carrying the CUP-I gene was sub-cloned in the single XbaI site of pLGSD5, to form recombinant plasmid pEHBII, containing the GALI0-CYCI promoter.

Brewing yeast strain NCYC240 was prepared for transformation with plasmid pEHBII by the following procedure. The yeast was grown in 100ml YEP medium (10g/litre yeast extract, 20g/litre peptone) supplemented with 2% w/v glucose to early exponential growth phase; cells were harvested and washed in cold sterile water prior to resuspending in 10mM Tris HCl buffer at pH7.6 containing IM Sorbitol, 10mM dithiothreitol and 40μg/ml Zymolyase 6000 (Kirin Brewery Co. Ltd) at 30°C. After 1.5 hours incubation the yeast cells are converted into spheroplasts which are harvested by centrifugation and washed three times in 20ml of a solution containing IM Sorbitol, 10mM CaCl₂, 10 mM Tris HCl at pH7.6. Spheroplasts are finally resuspended in Iml of a solution of IM Sorbitol, 10mM CaCl₂, 10mM Tris HCl at pH7.6 and 100μℓ is added to 15μℓ of plasmid pEHBII. This mixture is incubated at room temperature for 30 minutes prior to the addition of Iml of a solution of 40% polyethylene gylcol 4000, 10mM CaCl₂, 10mM Tris HCl at pH7.6. After 2-5 minutes spheroplasts are harvested by centrifugation and gently resuspended in 0.5ml NEP Sorbitol medium (MgSO₄ 7H₂O 2g/ℓ, (NH₄)₂ SO₄ 2g/ℓ, KH₂PO₄ 3g/ℓ, CaCl₂ 2H₂0 0.25g/ℓ, yeast extract 2g/ℓ, peptone 3g/ℓ glucose 40g/ℓ, IM sorbitol) and incubated at 28°C for I hour before plating in molten NEP Sorbitol supplemented with 3% w/v agar and 0.2mM CuSO₄.7H₂O. Plates were incubated for 4-6 days at 28°C after which time copper resistance colonies were picked and transferred to NEP supplemented with 2% agar and 0.2mM CuSO₄.7H₂0. Transformants of NCYC240 harbouring the plasmid pEHBII were verified by their ability to grow on NEP agar containing ImM CuSO₄.7H₂0. In addition, these transformants could be detected by growth on M63 medium containing galactose and the chromogenic indicator Xgal (5-bromo-4-chloro-3-indolyl-beta-D-galactoside) according to the procedure of Guarente (1983, in "Methods in Enzymology, Recombinant DNA, Vol. 107, eds. Wu, R. and Grossman, L., p. 181). Yeast colonies producing beta-galactosidase (harbouring peHBII) develop a blue/green colour which is not apparent in those colonies lacking pEHBII. Thus it can be shown by means of a simple plate assay that brewing yeast transformed with the plasmid pEHBII is capable of producing enzymatically active beta-galactosidase.

The yeast strain NCYC 240 (pEHBII) has been deposited at the National Collection of Yeast Cultures, Colney Lane, Norwich NR4 7AU, United Kingdom on December 12th 1984 under No. NCYC 1547.

Induction of beta-galactosidase production in brewing yeast

Brewing yeast strains NCYC240 (pEHBII) and NCYC240 (pETI3:I) described in the aforesaid Patent Application were grown in aerobic shake flask culture (NEP medium plus 2% glucose and 0.2mM CuSO₄. 7H₂O) at 28°C. Late stationary phase cells were harvested from the culture medium and assayed for beta-galactosidase activity as described by Miller (In "Experiments in Molecular Genetics" ed. Miller, J.H., Cold Spring Harbor Laboratory, Cold Spring Harbor, New York, 1972).

Cells were washed × 2 in distilled water and resuspended in Z buffer (16.lg/l $Na_2HPO_4$ $7H_2O$, 5.5g/l $NaH_2PO_4$. $H_2O$, 0.75 g/l KCl, 0.246 g/l $MgSO_4$. $7H_2O$, 2.7 ml/l 2 - mercaptoethanol). The absorbance at 600nm was determined and the cells were permeabilized by the addition of 3 drops of chloroform, 2 drops of 0.l% sodium dodecyl sulphate (SDS) and vortexing for l0 sec. To l ml of permeabilized cells in Z buffer was added 0.2 ml of O-nitrophenol-galactoside (ONPG) (4 mg/ml in phosphate buffer, pH 7) and the reaction mixture was incubated at 28°C. The reaction was stopped by the addition of 0.5 ml lM $Na_2CO_3$, cell debris removed, and the absorbance at 420 nm determined. Beta-Galactosidase units were calculated as described by Miller (1972).

Assays revealed that NCYC 240 (pETl3:l) did not possess any endogenous beta-galactosidase activity, whereas NCYC 240 (pEHBll) produced approximately 1.05 units of beta-galactosidase. When cells of NCYC 240 (pEHBll) were resuspended in 2 volumes of 2% galactose solution and incubated at 28°C for a prolonged period of time, beta-galactosidase activity was induced to a considerably higher level. Thus, after five hours induction, l35 units of beta-galactosidase were obtained, without any increase in cell number, whereas after 23 hours induction and a 2 fold increase in cell number 4l54 units of beta-galactosidase were obtained. When either strain NCYC 240 or NCYC 240 (peTl3:l) were subjected to the same treatment there was no detectable beta-galactosidase.

Galactose induction of beta-galactosidase production in brewing yeast following a beer fermentation

Plasmid pEHBll was transformed, as described previously, into a proprietary strain of lager yeast, BB10.1/BB10.1 (pEHB11) and NCYC 240 (peHBll) were grown in NEP medium plus 2% glucose and 0.2mM $CuSO_4.7H_2O$ at 28°C, yeast were harvested and pitched (inoculated) into air saturated brewers wort. Fermentation proceeded anaerobically at l2°C until there was no further reduction in the specific gravity of the beer. Fermentation profiles were closely monitored and direct comparisons were made with fermentations performed on the same wort with the parental brewing yeast, NCYC 240 and BBl0.l which had not been transformed with plasmid PEHBll. In all cases the genetically modified yeast fermented the wort at the same rate and to the same degree of attenuation as the parental yeast strains. BBl0.l (PEHBll) and NCYC 240 (PEHBll) were harvested from their respective beers by centrifugation and following two washes in water were assayed for beta-galactosidase activity. Neither yeast strain produced in excess of 1.0 units of beta-galactosidase when assayed in this manner. However, following resuspension in 2% w/v galactose significant quantities of beta-galactosidase were obtained. Optimal conditions for galactose induction of beta-galactosidase activity were defined as follows: (i) 2-3% w/v galactose, (ii) minimal salts medium (1.7 g/l yeast nitrogen base without amino acids and ammonium sulphate, 5 g/l ammonium sulphate, 2.5% w/v casamino acids), (iii) cell density of 0.1 to 30%, preferably 10%, w/v (wet yeast pellet/induction medium) (iv) presence of molecular oxygen. Employment of these conditions resulted in the routine production of 2000-l200 units of beta-galactosidase in NCYC 240 (pEHBll) and BB 10.l (pEHBll) following 24 hours residence in induction medium.

Strain NCYC 240 (peHBll) was further evaluated under beer production conditions. In this case the yeast was used to ferment an all malt ale wort in a five-barrel (5 × 163.7 litres) experimental brewery. The beer produced was conditioned, fined and packaged into bottle, after which it was compared with a control beer fermented by the parental yeast NCYC 240, produced under identical conditions. Qualitative organoleptic analysis did not reveal any significant difference between the two products. Thus it is apparent that brewing yeast can be successfully genetically modified such that they are capable of producing significant quantities of heterologous protein following a post-fermentation induction, without adversely influencing the ability of the yeast to produce the primary beer product.

EXAMPLE II

Production of Human Serum Albumin protein in Brewing Yeast

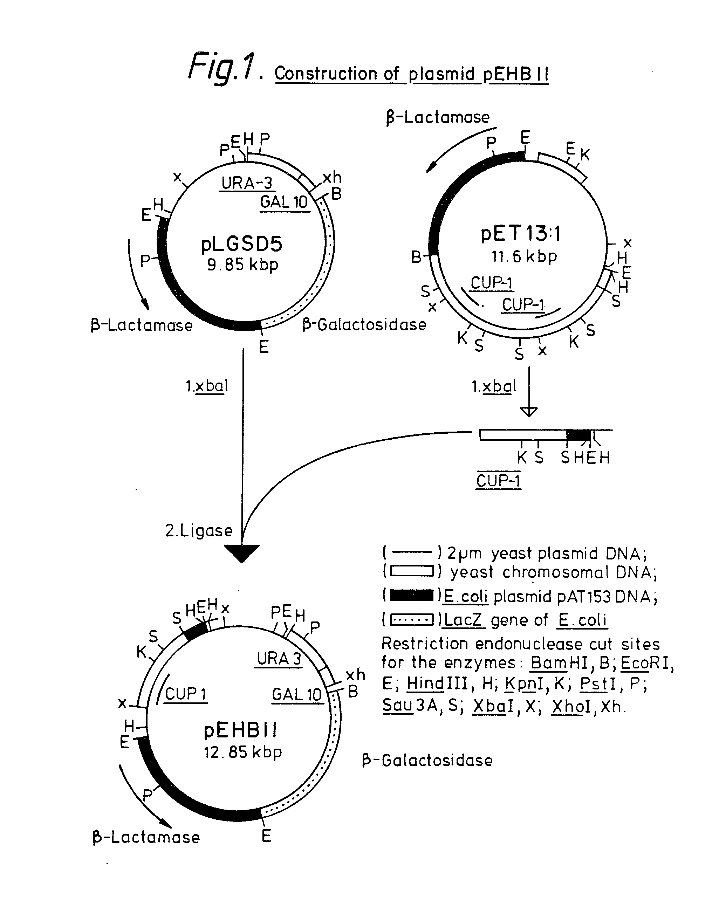

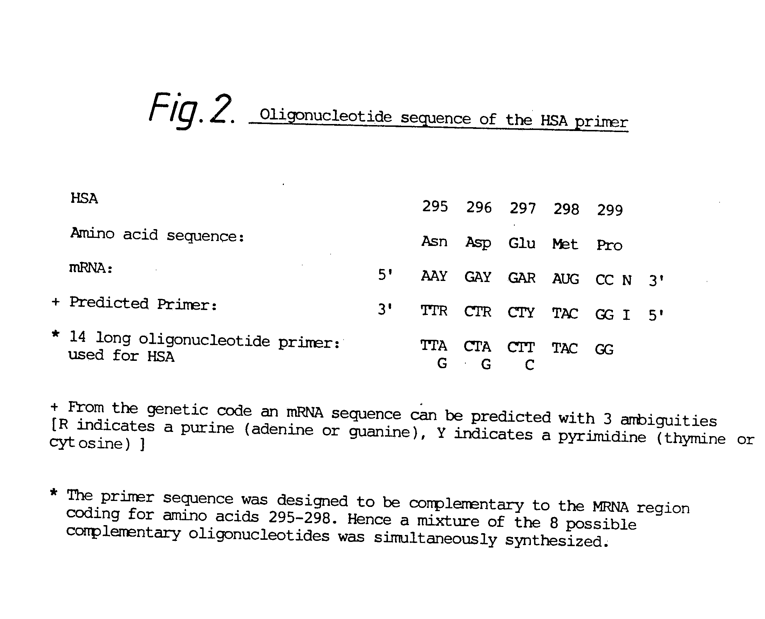

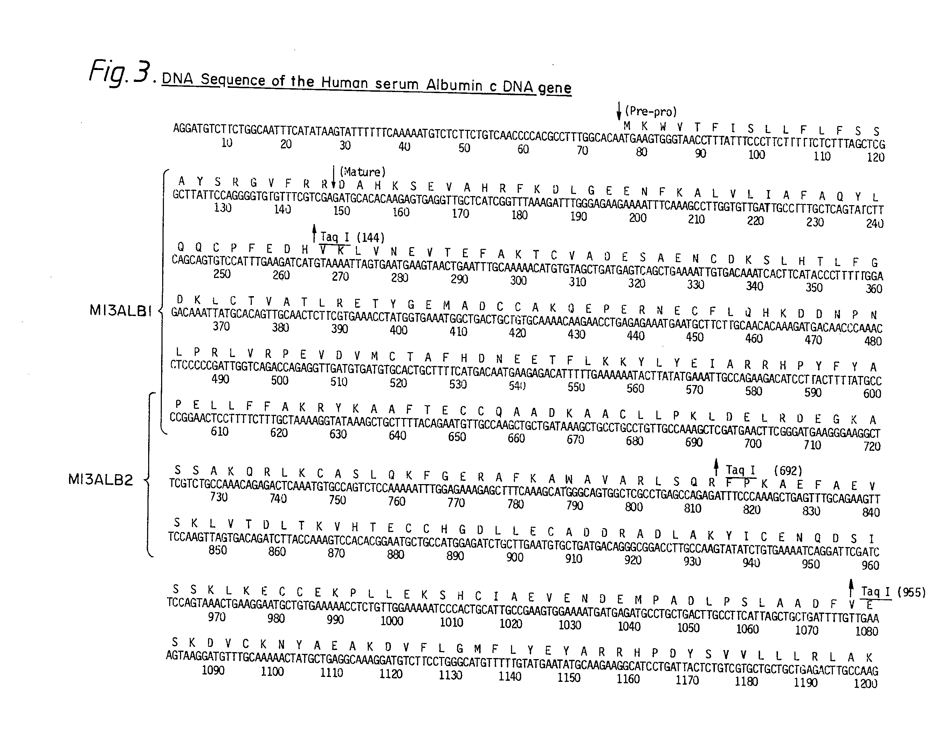

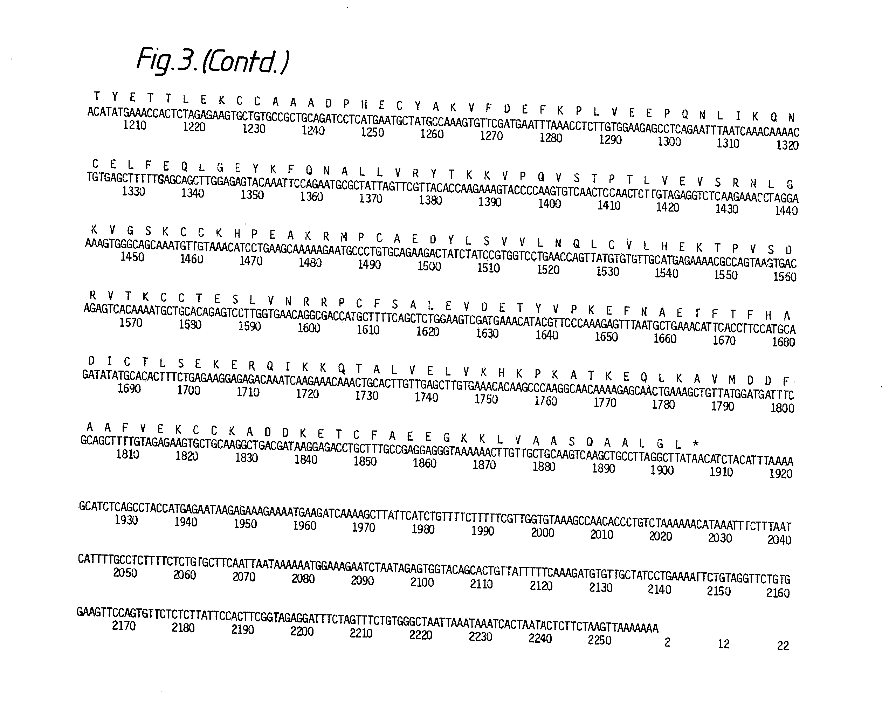

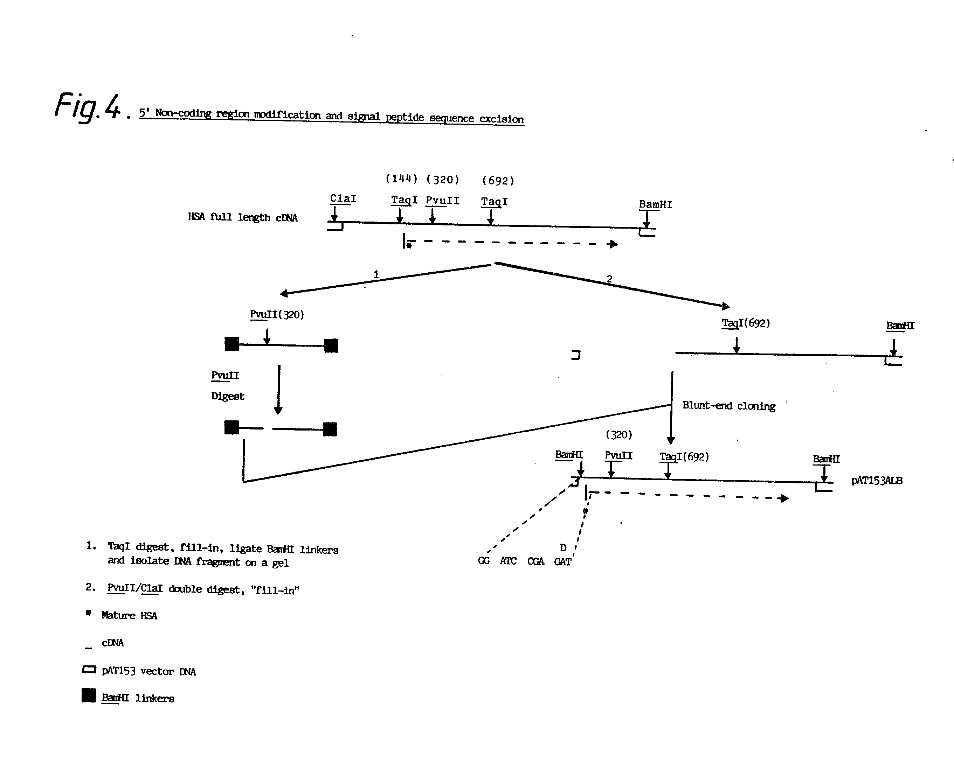

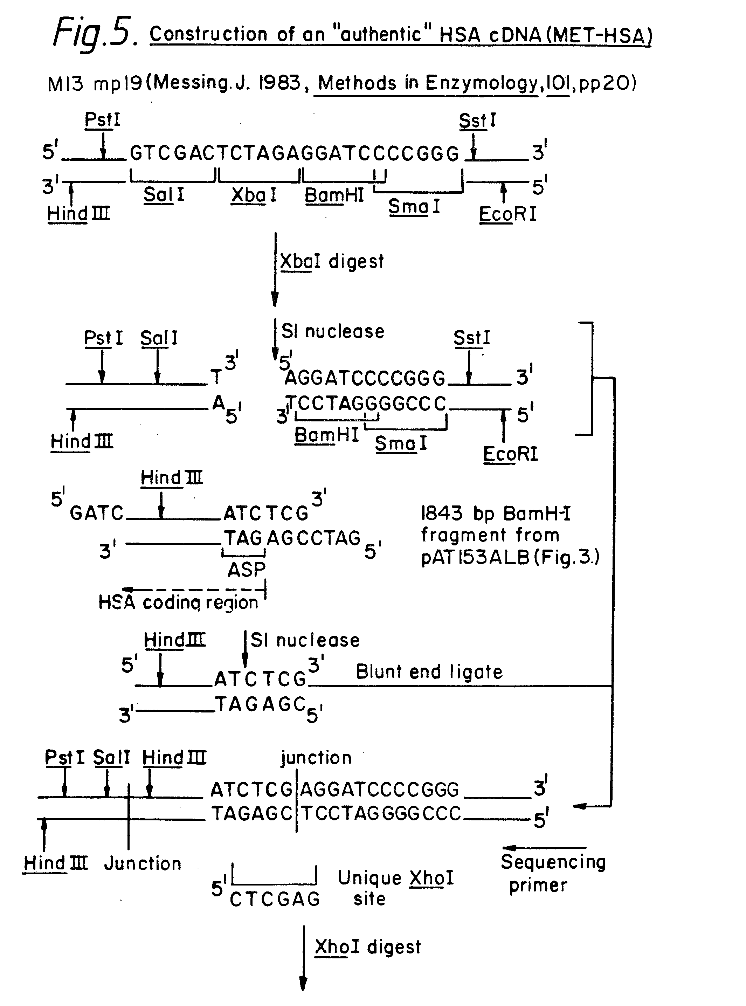

The cloning of the Human Serum Albumin (HSA) cDNA was carried out using a specific oligonucleotide primer (see Baralle, F.E., 1977, Cell, l0, 549-558: Noyes, B.E. etal, 1979, Proceedings of the National Academy of Science, U.S.A, 76, l770-l774; Hudson, P. etal, 1981, Nature, 29l, l27-l3l). By selecting a favourable region of the known amino acid sequence of the HSA protein (Dayhoff, M.O., 1976, Atlas of Protein Sequences and Structures Nat. Biomed. Res. Foundation Washington) it was possible to predict from the genetic code eight l4 long oligonucleotides, one of which should be exactly complementary to the HSA messenger RNA (mRNA) (see Figure 2 of the accompanying drawings). The oligonucleotides were synthesized simultaneously (by the method of Wallace, R.B., 1981, Nucleic Acids Research, 9, 879-894) using the solid phase phosphotriester method developed by Gait, M.K., etal, (1980, Nucleic Acids Research, 8, l08l-l096). This mixture of synthetic oligonucleotides was used as a primer for cDNA synthesis using human liver RNA as a template (see Baralle, F.E., 1977, Cell, l2, l085-l095). The synthesized cDNA was fractionated on a denaturing gel and an appropriately sized band was eluted. This single stranded cDNA was then converted to double strand by the 'loop back' reaction with Klenow DNA polymerase (see Wickens, M.P., etal, 1978, Journal of Biological Chemistry, 253, 2483-2495). The resulting double stranded cDNA was digested with Taql and ligated to Ml3mp9 vector which had been AccI-restricted and alkaline phosphatase-treated and the ligation mixture was transformed into the E.coli strain JMl0l (Messing J. and Vieira, J., 1981, Analects, 9. (8), l). The DNA sequence analysis (determined by the method of Sanger, F. etal, 1980, Journal of Molecular Biology, 143, 161-178) of a sample of the recombinant phage allowed the identification of two clones herein designated MI3ALBI and MI3ALB2 (Fig. 3). These clones included the DNA sequence of the HSA cDNA between nucleotides 144-692 (MI3ALBI) and 692-955 (MI3ALB2) (Fig. 3).

A cDNA library was prepared synthesizing cDNA with total liver mRNA as template and using oligo thymine dTI2-I8 as primer. The cDNA's were converted to double strands as described previously and the single strand hairpin loop structure was digested with SI nuclease (Efstratiadis, A. etal, 1976, Cell, 7, 279-288). The products were purified on a Sephacryl S 300 column and the eventual 5′ overhanging ends of the cDNA were repaired by "filling in" with the Klenow fragment of DNA polymerase I. The cDNA molecules at this stage were predominantly blunt ended so they were ligated to the PvuII site of pATI53PvuII8 (previously treated with alkaline phosphatase to avoid its circularization (Anson, D. et.al. The EMBO Journal, 3, 1984, pp 1053-1064) and transformed into E.coli strain MCI06I (Casadaban, M.T., & Cohen, SN., 1980 Journal of Molecular Biology, 138, 179-207) or another appropriate strain. Approximately 10,000 recombinant colonies were produced from 10 μ g of mRNA. The DNA in these colonies was screened for HSA DNA sequences using radioactive probes prepared from MI3ALBI and MIALB2 by nick translation (Rigby, P.W.J. etal, 1977, Journal of Molecular Biology, 113, 237-251). Colonies of E.coli strain MCI06I harbouring recombinant cDNA clones possessing DNA sequence homology with the HSA gene were isolated as described by Baralle, F.E. etal (1980, Nucleic Acids Research, 8, 4393-4404). Full length cDNA clones were characterised by DNA sequence analysis (Maxam, A.M. and Gilbert, W., 1980, Methods in Enzymology, 65, 499-560; Sanger, F. etal, 1980, Journal of Molecular Biology, 143, 161-178) and were found to include the entire HSA cDNA gene (Fig. 3). The 5′ non-coding region and the signal peptide sequence of the full lengths HSA cDNA were excised in the way shown in Figure 4 to produce plasmid pATI35ALB.

### Construction of a MET-HSA cDNA

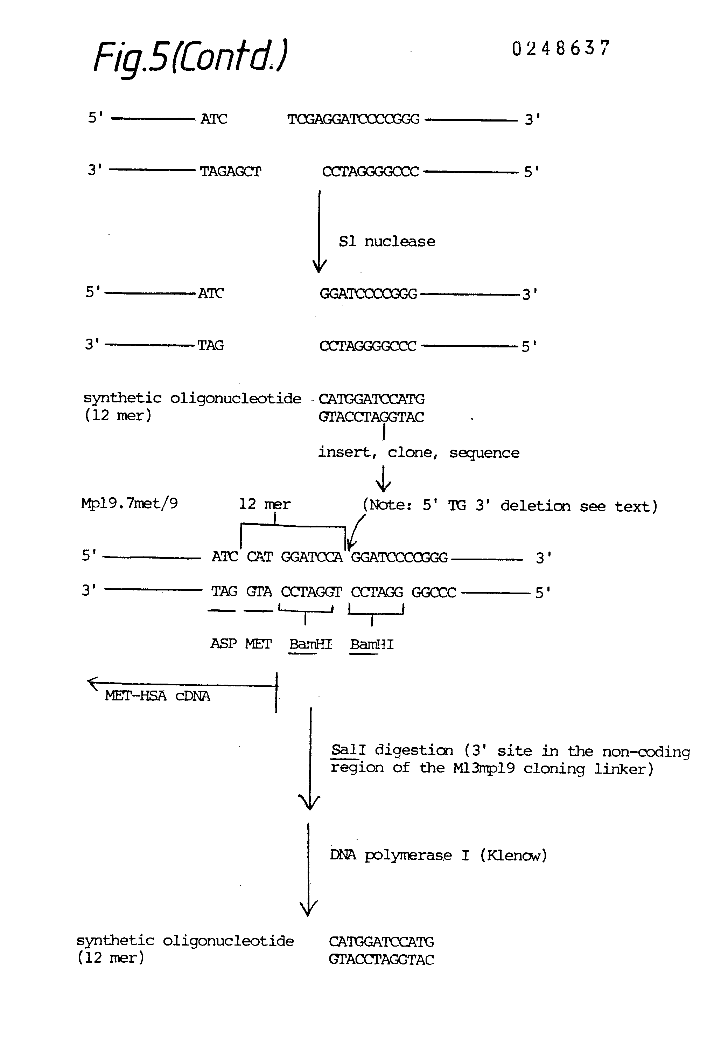

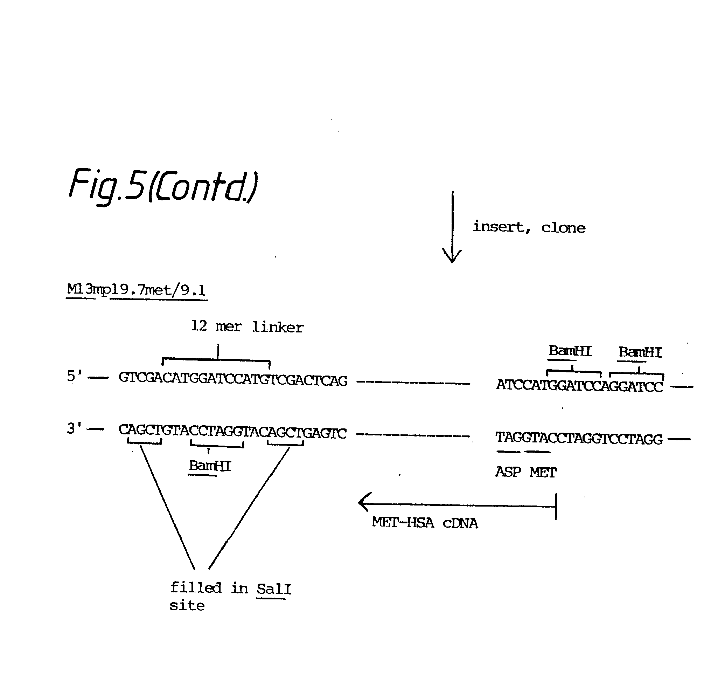

The strategy for the construction of a MET-HSA cDNA employed generally known techniques: a summary is shown in Figure 5. The 1843 base pair BamHI fragment from pATI53 ALB was isolated, and made flush ended by nuclease SI treatment (Maniatis et al, 1982, Molecular Cloning: A Laboratory Manual, Cold Spring Harbor, USA) at the XbaI site of MI3mpI9 (Messing, 1983, Methods in Enzymology, Recombinant, 101, 20-78), the latter having been similarly made flush ended by nuclease SI treatment. Following transfection of E. coli JMI0I, lysates were prepared from several plaques and single stranded (SS) DNA isolated from the mature bacteriophage particles. Subsequent to DNA sequence analysis (Sanger, 1977 et al, Proc. Nat. Acad. Sci., USA, 74, 5463) the recombinant MI3mpI9.7 was identified. Double stranded (RF) DNA was prepared and cleaved at the newly created unique XhoI site by treatment with the latter restriction endonuclease, followed by SI nuclease digestion to remove the 5′ single strand extensions.

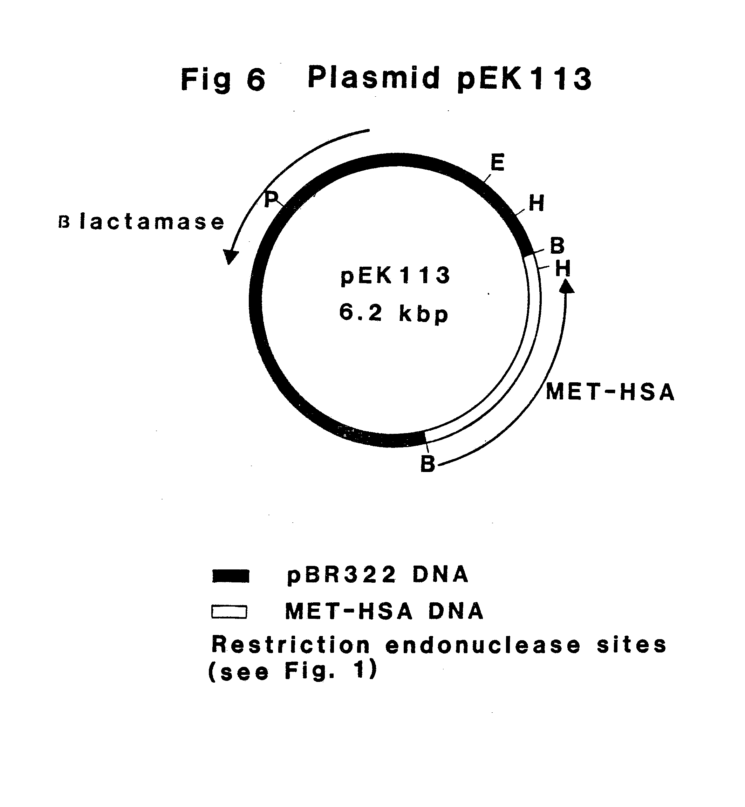

These manipulations served to expose the first codon (GAT) for the mature, native polypeptide sequence. Next, a synthetic oligonucleotide whose 5′ ends were unphosphorylated was inserted at the modified XhoI site. As previously, recombinants were analysed directly by DNA sequence determination following transfection and SS DNA preparation. The sequence of the relevant portions of one such recombinant, MI3 mp I9.7 met/9, is shown (Figure 5). This deviates from the expected sequence, also shown in Figure 5; this deviation is most probably due to secondary structure formation about the double BamHI site and subsequent excision of the non-bonded regions of the intrastrand partial duplex. These deviations are inconsequential since the sole purpose for the linker insertion was to introduce an initiation codon (ATG) and a BamHI site immediately upstream from the HSA coding sequence, which was achieved. For convenience a further BamHI site was introduced 3′ to the MET-HSA coding sequence in MI3mpI9.7 met/9. In this instance RF DNA of the latter molecule was cleaved at the unique SalI site, originating in the cloning linker of MI3mpI9, and this species made flush ended by treatment with E.coli DNA polymerase I (the large fragment or "Klenow" fragment (Maniatis et al, 1982, Molecular Cloning: A Laboratory Manual, Cold Spring Harbor, USA)). The same linker used above was inserted at this modified site. Following RF DNA preparation and BamHI digestion a suitable recombinant clone was identified and this was designated MI3mpI9.7 met/9.1 A single BamHI fragment carrying the full length MET-HSA cDNA gene was subsequently cloned in the single BamHI site of the E.coli vector pBR322 (Bolivar et al, 1977, Gene, 2, 95-113) to derive a plasmid designated pEKII3 (Figure 6). Plasmid pEKII3 was transformed into the E.coli strain MCI06I (Casadaban & Cohen, 1980, Journal of Molecular Biology, 138, 179-207) which was deposited in the National Collection of Industrial Bacteria (NCIB) Torry Research Station, PO Box 31, 135, Abbey Road, Aberdeen, Scotland, AB9 8DG on April 3rd 1986 and has the accession number NCIB 12242.

### The Inducible Expression of HSA-like Protein in Brewing Yeast

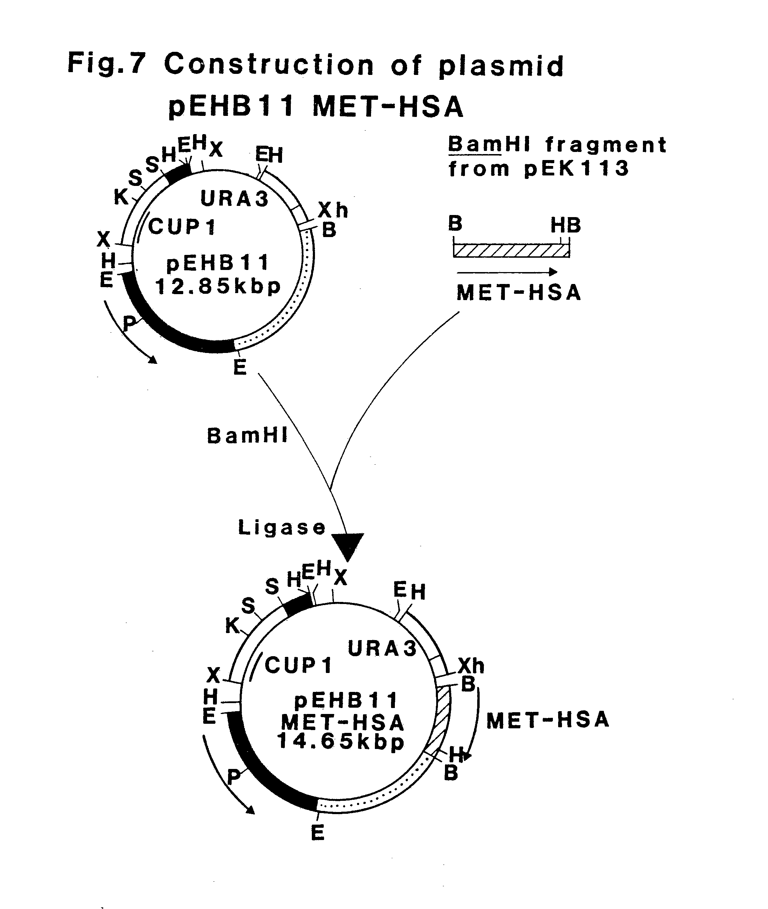

In order to produce HSA-like protein in brewing yeast by an inducible gene expression system it is necessary to fuse the MET-HSA cDNA coding sequence to an appropriate expression signal. The GALI0-CYCI hybrid promoter which controls the galactose inducible expression of the E.coli lacZ gene (β-galactosidase production) has general application as a system for mediating the galactose inducible expression of heteroloqous genes in brewing yeast. To take advantage of this, the MET-HSA cDNA is fused to the GALI0-CYCI promoter present in plasmid peHBII. Plasmid peHBII is digested with the restriction endonuclease BamHI and the BamHI fragment carrying the MET-HSA cDNA from pEKII3 is inserted. The orientation of insertion is determined by characterising the recombinant plasmids generated with restriction endonucleases and separating the fragments thus formed by agarose gel electrophoresis. A recombinant plasmid is isolated in which the 5' MET-HSA cDNA has fused with the 3' GALI0-CYCI hybrid promoter, such that following DNA sequence analysis (Maxam, A. M. and Gilbert, W., 1980, Methods in Enzymology, 65, 499-560) the junction of the fusion has the DNA sequence 5' ... TTAATA ATG ACC GGA TCC ATG GAT ... 3' (See Figure 7). This plasmid is designated peHBII-MET-HSA and may be introduced into brewing yeast strains NCYC 240 and BBI0.I by transformation and selection for copper resistance as described previously. Brewing yeast strains transformed with plasmid peHBII-MET-HSA are expected to produce under appropriate conditions an HSA-like protein consisting of five additional amino acids prior to the first N terminal amino acid of mature HSA (N-methionine, threonine, glycine, serine, methionine-HSA-C).

Strain NCYC 240 and BBI0.I harbouring peHBII-MET-HSA may be grown to stationary phase in NEP glucose medium supplemented with 0.2mM CuSO$_4$.7H$_2$0. Cells are then harvested by centrifugation and washed in water before resuspending for 24 hours under optimal conditions for galactose induction of gene expression, described previously. Crude cell extracts of induced (plus galactose) and uninduced (without galactose) cultures are prepared by disrupting the cells on a Braun homogeniser in 0.IM sodium phosphate pH 7. 5, ImM phenylmethylsulfonyl fluoride and 5mM 2-mercaptoethanol. The resultant cell extracts are assayed for the presence of Human Serum Albumin protein by SDS: polyacrylamide gel electrophoresis followed by Western blotting (see Mellor, J. et al, 1985, Gene, 33, p2l5). The results of this assay indicate that those cultures which had been induced in the presence of galactose contained significant quantities of intracellular Human Serum Albumin-like protein, whereas uninduced cultures do not.

A similar galactose induction of Human Serum Albumin-like protein production can be obtained with both NCYC 240 (peHBII-MET-HSA) and BBI0.I (peHBII-MET-HSA) harvested from a beer fermentation in which the yeast strain has first been used to ferment brewers wort under anaerobic fermentative conditions. Yeast harvested at the end of the beer fermentation does not contain detectable quantities of Human Serum Albumin-like protein, whereas following 24 hours of galactose induction significant amounts of protein can be detected. It is further noted that brewing yeasts generally modified in the manner described are capable of fermenting brewers wort at the same rate and to the same degree of attenuation as their unmodified parental counterparts; beers thus produced are indistinguishable from controls or organoleptic analysis.

## The Inducible Expression of MET-HSA in Brewing Yeast

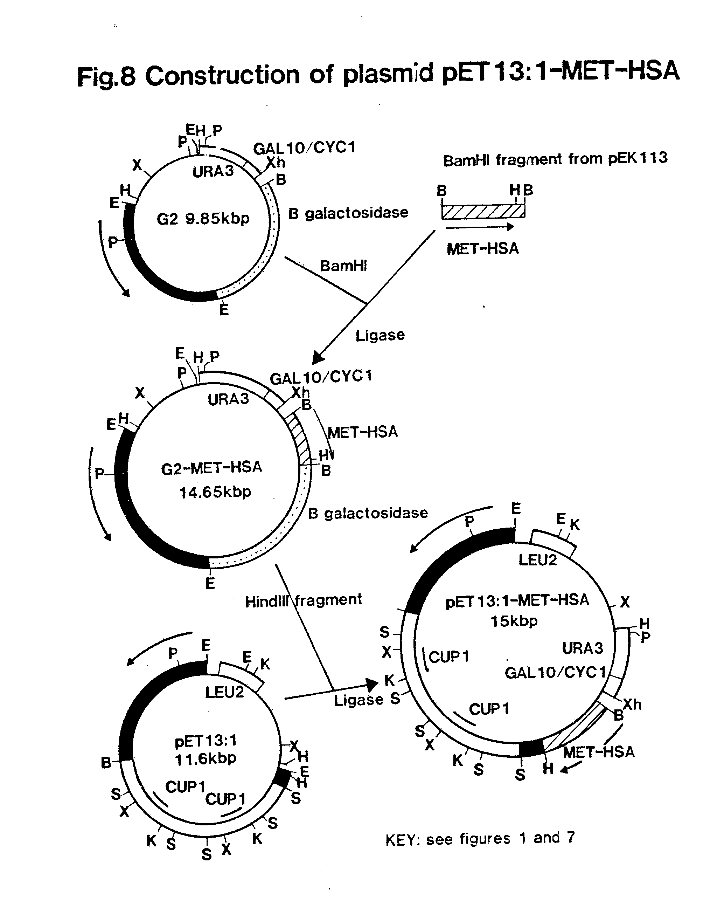

The GALI0-CYCI-HSA fusion plasmid pEHBII-MAT-HSA can be shown to produce an HSA-like protein in brewing yeast following post-fermentation induction in galactose induction medium. this protein is described as HSA-like since it possesses five extra amino acids at the N-terminus, as described previously. In order to produce a more "authentic" HSA protein it is necessary to construct a GALI0-CYCI hybrid promoter transcriptional fusion with the MET-HSA cDNA in which the 5' translational ATG codon (methionine) is supplied by the MET-HSA cDNA. For this purpose the ATG− GALI0-CYCI promoter carried by the publicly available plasmid G2 can be used (Guarente, 1983, Methods in Enzymology, Recombinant DNA, I0I, I8I-I9I). Plasmid G2 is digested with the restriction endonuclease BamHI and ligated with 1.8 kilo base-pair MET-HSA cDNABamHI fragment from pEKII3. The orientation of insertion is confirmed by restriction endonuclease indigestion and shows that the 5' MET-HSA cDNA is fused to the 3' GALI0-CYC ATG− promoter. This plasmid is designated G2 MET-HSA (see Figure 8). Plasmid G2 MET-HSA carries an E.coli plasmid origin of DNA replication, the β-lactamase gene conferring ampicillin resistance in E.coli, the 2µm origin of DNA replication of yeast, the URA-3 gene of yeast (facilitating selection by complementation of ura-3 auxotrophs in laboratory yeast) and the GALI0-CYCI-MET-HSA fusion cassette. Transcription of the MET-HSA gene is expected to result in the synthesis of a messenger RNA which will initiate translation at the first ATG in the message, and this ATG is provided by the MET-HSA gene.

In order to regulate the expression of the MET-HSA gene in brewing yeast, and thus the production of an "authentic" recombinant Human Serum Albumin protein, it is necessary to fuse the GALI0-CYCI-MET-HSA expression unit to the CUP-I gene. This is accomplished by digesting plasmid G2-MET-HSA with the restriction endonuclease HindIII, which cleaves upstream (5') of the URA-3 gene and downstream (3') of the MET-HSA coding sequence (see Figure 8), and subcloning this fragment into the HindIII site of pETI3:I to form plasmid designated pETI3:I-MET-HSA (Figure 8). A second plasmid, designated pEKI0I4 ALBI, with similar features to pETI3:I-MET-HSA, is shown in Figure I0.

Plasmid pETI3:I-MET-HSA is subsequently transformed into the brewing yeast strains NCYC 240 and BBI0.I by the methods described previously. Brewing yeast strains harbouring plasmid pETI3:I-MET-HSA are grown to stationary phase in NEP glucose medium supplemented with 0.2mM CuSO$_4$.7H$_2$0. Cells are harvested and processed prior to the galactose induction of gene expression, as described previously. Cell extracts were prepared and assayed for the presence of N-methionyl human serum albumin, as described previously. In all cased brewing yeast strains harbouring the GALI0-CYCI-MET-HSA (pETI3:I-MET-HSA) produced significant quantities of N-methionyl human serum albumin protein. When the same strains are grown on brewers wort under anaerobic conditions, followed by induction of gene expression and HSA assay as described previously, significant quantities of N-methionyl human serum albumin can be detected. The yeast harvested at the end of beer fermentation does not possess any detectable N-methionyl human serum albumin protein prior to galactose induction.

## EXAMPLE III

Galactose induction of heterologous protein synthesis in the presence of glucose

Brewing yeast NCYC240, BB6 and BBI0.I were transformed to copper resistance with plasmids pEHBII and pETI3:I MET-HSA. Transformants were checked and verified as described (Hinchliffe & Daubney, Journal of the American Society of Brewing Chemists, 1986, 44, pp 98-I0I), The genetically modified yeasts harbouring each of the aforementioned plasmids were grown in NEP glucose medium supplemented with 0.2 mM $CuSO_4.7H_2O$, prior to inoculation into hopped brewers wort. Fermentations were performed under standard beer fermentation conditions, following which yeasts were separated from the beer by centrifugation. The residual yeast was then resuspended at 5% w/v cell density in minimal medium containing 2% w/v galactose (Sigma Chemical Co., G-0750, containing less than 0.0I% glucose) and 2.5% w/v hydrolysed casein. The yeast was stirred vigorously to ensure good mixing and ingression of oxygen. After five to twenty four hours of induction, samples of yeast were assayed for the presence of intracellular heterologous protein. Brewing yeast harbouring plasmid pEHBII gave rise to significant quantities of β-galactosidase, whereas brewing yeast harbouring pETI3:I did not produce any β-galactosidase. A comparison of the absolute level of intracellular β-galactosidase present in yeast induced in the presence or absence of glucose indicated that there was no significant difference in the relative amounts of enzyme produced. Whilst slight variation was observed between the rate at which β-galactosidase was synthesized between yeast induced in the presence and absence of glucose, the absolute levels of steady state protein were the same following twenty four hours induction. A similar result was observed for brewing yeast harbouring plasmid pETI3:I MET-HSA induced under similar conditions.

These results indicate that glucose does not adversely influence the absolute amount of protein synthesized when yeast is induced in the presence of both galactose and glucose.

## EXAMPLE IV

Galactose induction of heterologous protein synthesis using commercial galactose containing equivalent concentrations of glucose.

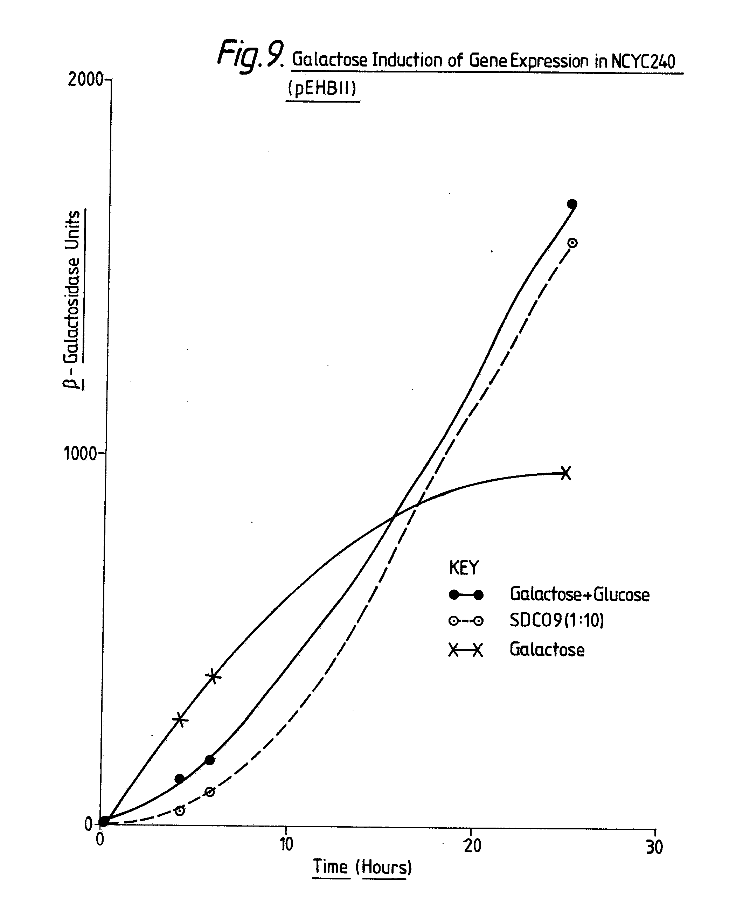

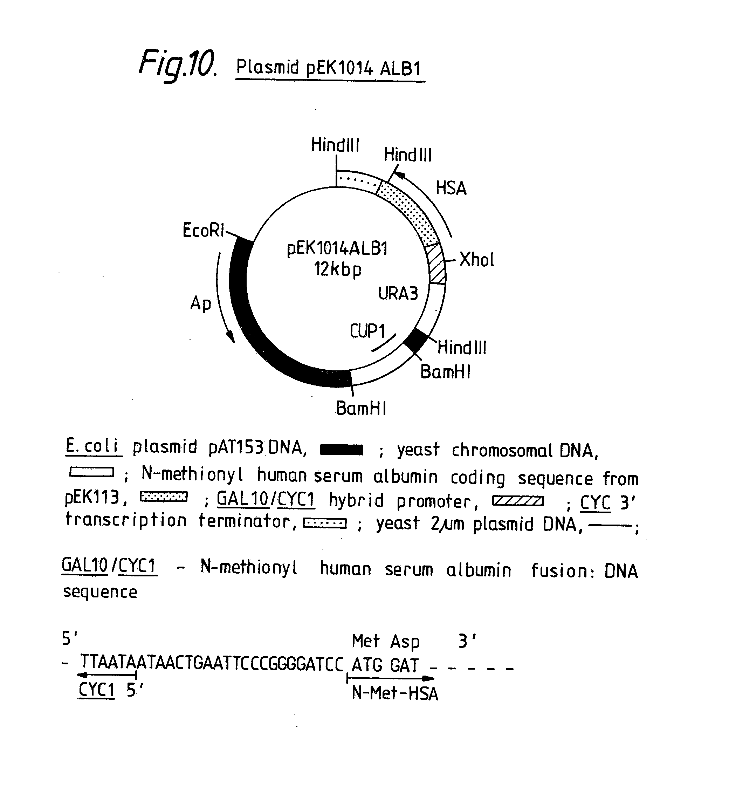

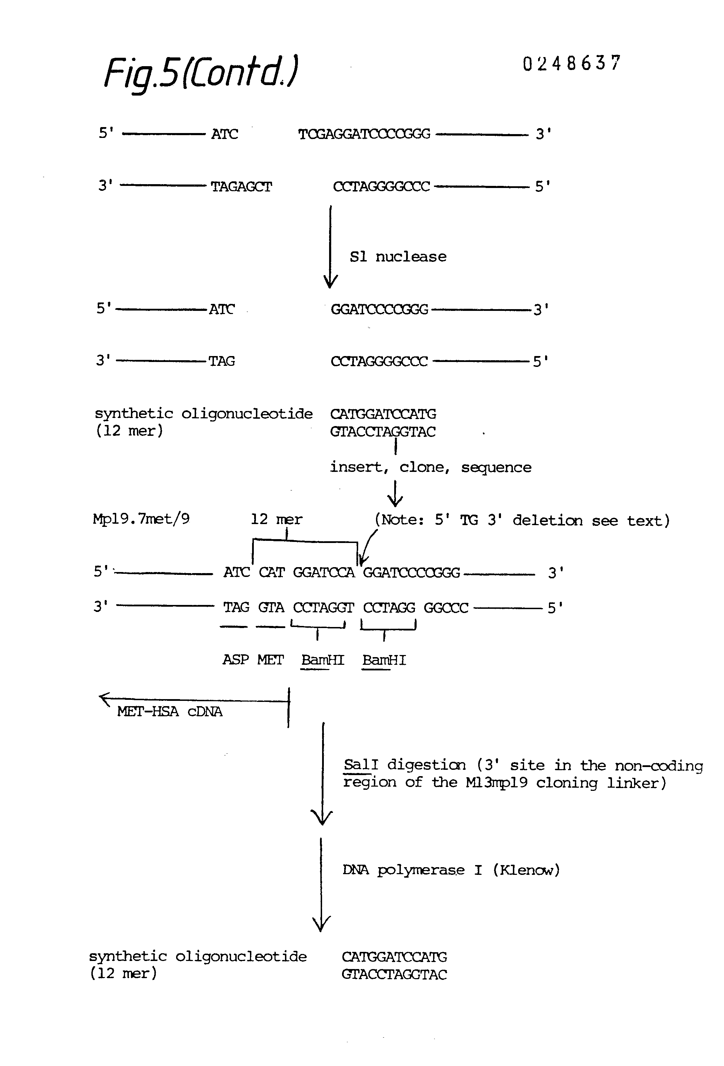

The galactose induction characteristics of the yeast described above were further evaluated in a hydrolysed lactose medium (SDC09) derived from the enzymic hydrolysis of cheese-whey permeates (Specialist Dairy Ingredients, Chichester House, Chichester Street, Chester). The hydrolysed lactose medium was diluted ten-fold in distilled water prior to inoculation at 5% w/v yeast with the genetically modified brewing yeast. The same yeast cultures were similarly induced in minimal medium containing 2% w/v galactose and 2.5% w/v hydrolysed casein as well as the same medium supplemented with 2% w/v glucose. Yeasts were induced over a twenty four hour period and samples taken to determine the amount of protein (β-galactosidase) present. The results presented in Figure 9 indicate that yeast harbouring plasmid pEHBII could be induced to synthesize similar absolute amounts of intracellular β-galactosidase following twenty four hours incubation in the presence of either diluted hydrolysed lactose of galactose plus glucose. In addition the results presented in Figure 9 suggest that higher absolute levels of β-galactosidase can be produced in NCYC240 (pEHBII) in the presence of galactose plus glucose, rather than galactose alone.

## EXAMPLE V

Galactose Induction of N-Methionyl-Human Serum Albumin Synthesis at High Cell Dry Weight by the Controlled Addition of Glucose and Galactose Mixtures

The brewing yeast strain BBII was transformed to copper resistance with the plasmid pEKI0I4 ALBI (fig.I0). Transformants were checked and verified as described previously (Hinchliffe and Daubney, 1986, Journal of the American Society of Brewing Chemists, 44, 98-I0I). The genetically modified brewing yeast containing the plasmid was grown in hopped brewers wort in an experimental brewery. Yeast was harvested by centrifugation and cells were resuspended at high cell dry weight (50g per litre) in I.25 litres (final volume) of L.B. medium (Miller, J.H., I972, In "Experiments in Molecular Genetics", Cold Spring Harbor Laboratory, Cold Spring Harbor, New York) in a Braun Biostat E fermenter. Fermentation conditions were set to 30°C, pH5.0+ 0.I, dissolved oxygen tension (DOT) ≥25% air saturation. Glucose and galactose were introduced to the fermenter by the gradual addition of 0.25 litres of a I5% w/v glucose, I5% w/v galactose solution over a 5 hour period. Intracellular N-methionyl human serum albumin was observed after two hours under these conditions, maximum levels were achieved after 3.5 hours as determined by densitometric scanning of a Coomassie blue stained SDS: polyacrylamide protein gel containing yeast cell extracts as described previously. During the course of this experiment the concentration of glucose and galactose were monitored in the fermenter using standard procedures. It was observed that there was no apparent accumulation of glucose during the fermentation since the glucose concentration always remained below the basal level of about 0.2g per litre; thus substantially no glucose was present under these conditions since presumably the yeast catabolized this sugar immediately upon addition. On the other hand galactose was seen to briefly accumulate at the beginning of the fermentation; however, after two hours the galactose concentration in the fermenter was substantially reduced with the concomittant appearance of intracellular N-methionyl human serum albumin. The overall process used only 50g per litre carbohydrate (25g per litre glucose and 25g per litre galactose) and yielded levels of intracellular N-methionyl human serum albumin equivalent to those obtained previously employing simple batch additions of glucose: galactose mixtures using the same genetically modified brewing yeast.

## Claims

1. A process for the production of a protein or peptide which is heterologous to yeast which comprises inducing the expression of the protein or peptide in a yeast which has been genetically modified to be capable of expressing the heterologous protein or peptide upon induction by galactose, the induction being carried out in the presence of a galactose-containing medium which contains a significant amount of glucose and obtaining the said heterologous protein or peptide therefrom.

2. A process according to claim 1 wherein the galactose-containing medium is derived from whey permeates or from hydrolysed raffinose.

3. A process according to claim 1 wherein the galactose-containing medium is derived from the enzymic and/or chemical hydrolysis of lactose.

4. A process according to any one of the preceding claims wherein the galactose-containing medium is added gradually to an aqueous medium containing the genetically modified yeast.

5. A process according to any one of the preceding claims wherein the protein or peptide is human serum albumin or a human serum albumin like protein.

6. a process according to any one of the preceding claims wherein the yeast used is a genetically engineered modification of an industrial strain of <u>Saccharromycescerevisiae</u> or <u>S. carlsbergensis</u>.

7. A process according to claim 6 wherein the yeast used is a genetically engineered modification of brewing yeast NCYC 240, BB6, BB10.1, or BB11.

8. A process according to any one of the preceding claims wherein the yeast is obtained from the product of a fermentation process which is carried out in an aqueous carbohydrate-containing medium under such conditions that the yeast multiplies with substantially no expression of the heterologous protein or peptide taking place.

9. A process according to claim 8 wherein the yeast is separated from the fermentation products prior to the expression of the heterologous protein or peptide.

10. A process according to claim 8 or 9 wherein the aqueous carbohydrate-containing medium contains maltose as the major sugar present.

11. A process according to claim 10 wherein the aqueous carbohydrate-containing medium is a barley malt-based beer wort.

12. A process according to any one of claims 8 to 11 wherein the fermentation is effected at 8 to 25°C.

13. A process according to any one of claims 8 to 10 wherein the aqueous carbohydrate-containing medium is a fermentation medium for the production of potable distilled ethanol or power ethanol.

14. A process according to claim 13 wherein the medium is based on grain, potatoes, cassava, sugar cane, sugar beet, or a lignocellulosic material optionally pretreated to convert the cellulose and/or starch therein into fermentable sugars.

15. A process according to any one of claims 8 to 14 wherein the fermentation is a substantially anaerobic fermentation.

*Fig.1.* Construction of plasmid pEHB II

β-Lactamase

pEHP
URA-3
GAL 10
x
E H
B
xh

**pLGSD5**
**9.85 kbp**

P

β-Lactamase

E

β-Galactosidase

---

β-Lactamase

P E
E K

**pET 13:1**
**11.6 kbp**

B

CUP-1
CUP-1

x
H
E
H
S

S
x
K S
S x
K S

---

1.xbal

1.xbal

K S    S H E H

CUP-1

2.Ligase

---

S H E H x
K S

CUP 1

x

H
E

**pEHB II**
**12.85 kbp**

P E H
P

URA 3

GAL 10

xh
B

P

β-Lactamase

E

β-Galactosidase

---

( —— ) 2 µm yeast plasmid DNA;
( ▭ ) yeast chromosomal DNA;
( ▬ ) E.coli plasmid pAT153 DNA;
( ⬚ ) LacZ gene of E.coli
Restriction endonuclease cut sites
for the enzymes: BamHI, B; EcoRI,
E; HindIII, H; KpnI, K; PstI, P;
Sau3A, S; XbaI, X; XhoI, Xh.

# Fig. 2. Oligonucleotide sequence of the HSA primer

| HSA | | 295 | 296 | 297 | 298 | 299 | |
|---|---|---|---|---|---|---|---|
| Amino acid sequence: | | Asn | Asp | Glu | Met | Pro | |
| mRNA: | 5' | AAY | GAY | GAR | AUG | CC N | 3' |
| + Predicted Primer: | 3' | TTR | CTR | CTY | TAC | GG I | 5' |
| * 14 long oligonucleotide primer: used for HSA | | TTA G | CTA G | CTT C | TAC | GG | |

+ From the genetic code an mRNA sequence can be predicted with 3 ambiguities [R indicates a purine (adenine or guanine), Y indicates a pyrimidine (thymine or cytosine) ]

* The primer sequence was designed to be complementary to the MRNA region coding for amino acids 295-298. Hence a mixture of the 8 possible complementary oligonucleotides was simultaneously synthesized.

# Fig. 3. DNA Sequence of the Human serum Albumin c DNA gene

↓ (Pre-pro)

```
                                                               M K W V T F I S L L F L F S S
AGGATGTCTTCTGGCAATTTCATATAAGTATTTTTTCAAAAATGTCTCTTCTGTCAACCCCACGCCTTTGGCACAATGAAGTGGGTAACCTTTATTTCCCTTCTTTTTCTCTTTAGCTCG
```

```
       | (Mature)
A Y S R G V F R R↓D A H K S E V A H R F K D L G E E N F K A L V L I A F A Q Y L
GCTTATTCCAGGGGTGTGTTTCGTCGAGATGCACACAAGAGTGAGGTTGCTCATCGGTTTAAAGATTTGGGAGAAGAAAATTTCAAAGCCTTGGTGTTGATTGCCTTTGCTCAGTATCTT
```

```
              ↑ Taq I (144)
Q Q C P F E D H V K L V N E V T E F A K T C V A D E S A E N C D K S L H T L F G
CAGCAGTGTCCATTTGAAGATCATGTAAAATTAGTGAATGAAGTAACTGAATTTGCAAAAACATGTGTAGCTGATGAGTCAGCTGAAAATTGTGACAAATCACTTCATACCCTTTTTGGA
```

MI3ALBI {
```
D K L C T V A T L R E T Y G E M A D C C A K Q E P E R N E C F L Q H K D D N P N
GACAAATTATGCACAGTTGCAACTCTTCGTGAAACCTATGGTGAAATGGCTGACTGCTGTGCAAAACAAGAACCTGAGAGAAATGAATGCTTCTTGCAACACAAAGATGACAACCCAAAC
```

```
L P R L V R P E V D V M C T A F H D N E E T F L K K Y L Y E I A R R H P Y F Y A
CTCCCCCGATTGGTCAGACCAGAGGTTGATGTGATGTGCACTGCTTTTCATGACAATGAAGAGACATTTTTGAAAAAATACTTATATGAAATTGCCAGAAGACATCCTTACTTTTATGCC
```

```
P E L L F F A K R Y K A A F T E C C Q A A D K A A C L L P K L D E L R D E G K A
CCGGAACTCCTTTTCTTTGCTAAAAGGTATAAAGCTGCTTTTACAGAATGTTGCCAAGCTGCTGATAAAGCTGCCTGCCTGTTGCCAAAGCTCGATGAACTTCGGGATGAAGGGAAGGCT
```

MI3ALB2 {
```
S S A K Q R L K C A S L Q K F G E R A F K A W A V A R L S Q R F P K A E F A E V
                                                                        ↑ Taq I (692)
TCGTCTGCCAAACAGAGACTCAAATGTGCCAGTCTCCAAAAATTTGGAGAAAGAGCTTTCAAAGCATGGGCAGTGGCTCGCCTGAGCCAGAGATTTCCCAAAGCTGAGTTTGCAGAAGTT
```

```
S K L V T D L T K V H T E C C H G D L L E C A D D R A D L A K Y I C E N Q D S I
TCCAAGTTAGTGACAGATCTTACCAAAGTCCACACGGAATGCTGCCATGGAGATCTGCTTGAATGTGCTGATGACAGGGCGGACCTTGCCAAGTATATCTGTGAAAATCAGGATTCGATC
```

```
S S K L K E C C E K P L L E K S H C I A E V E N D E M P A D L P S L A A D F V E
                                                                              ↑ Taq I (955)
TCCAGTAAACTGAAGGAATGCTGTGAAAAACCTCTGTTGGAAAAATCCCACTGCATTGCCGAAGTGGAAAATGATGAGATGCCTGCTGACTTGCCTTCATTAGCTGCTGATTTTGTTGAA
```

```
S K D V C K N Y A E A K D V F L G M F L Y E Y A R R H P D Y S V V L L L R L A K
AGTAAGGATGTTTGCAAAAACTATGCTGAGGCAAAGGATGTCTTCCTGGGCATGTTTTTGTATGAATATGCAAGAAGGCATCCTGATTACTCTGTCGTGCTGCTGCTGAGACTTGCCAAG
```

*Fig.3.(Contd.)*

```
    T Y E T T L E K C C A A A D P H E C Y A K V F D E F K P L V E E P Q N L I K Q N
ACATATGAAACCACTCTAGAGAAGTGCTGTGCCGCTGCAGATCCTCATGAATGCTATGCCAAAGTGTTCGATGAATTTAAACCTCTTGTGGAAGAGCCTCAGAATTTAATCAAACAAAAC
    1210      1220      1230      1240      1250      1260      1270      1280      1290      1300      1310      1320

    C E L F E Q L G E Y K F Q N A L L V R Y T K K V P Q V S T P T L V E V S R N L G
TGTGAGCTTTTTGAGCAGCTTGGAGAGTACAAATTCCAGAATGCGCTATTAGTTCGTTACACCAAGAAAGTACCCCAAGTGTCAACTCCAACTCTTGTAGAGGTCTCAAGAAACCTAGGA
    1330      1340      1350      1360      1370      1380      1390      1400      1410      1420      1430      1440

    K V G S K C C K H P E A K R M P C A E D Y L S V V L N Q L C V L H E K T P V S D
AAAGTGGGCAGCAAATGTTGTAAACATCCTGAAGCAAAAAGAATGCCCTGTGCAGAAGACTATCTATCCGTGGTCCTGAACCAGTTATGTGTGTTGCATGAGAAAACGCCAGTAAGTGAC
    1450      1460      1470      1480      1490      1500      1510      1520      1530      1540      1550      1560

    R V T K C C T E S L V N R R P C F S A L E V D E T Y V P K E F N A E T F T F H A
AGAGTCACAAAATGCTGCACAGAGTCCTTGGTGAACAGGCGACCATGCTTTTCAGCTCTGGAAGTCGATGAAACATACGTTCCCAAAGAGTTTAATGCTGAAACATTCACCTTCCATGCA
    1570      1580      1590      1600      1610      1620      1630      1640      1650      1660      1670      1680

    D I C T L S E K E R Q I K K Q T A L V E L V K H K P K A T K E Q L K A V M D D F
GATATATGCACACTTTCTGAGAAGGAGAGACAAATCAAGAAACAAACTGCACTTGTTGAGCTTGTGAAACACAAGCCCAAGGCAACAAAAGAGCAACTGAAAGCTGTTATGGATGATTTC
    1690      1700      1710      1720      1730      1740      1750      1760      1770      1780      1790      1800

    A A F V E K C C K A D D K E T C F A E E G K K L V A A S Q A A L G L *
GCAGCTTTTGTAGAGAAGTGCTGCAAGGCTGACGATAAGGAGACCTGCTTTGCCGAGGAGGGTAAAAAACTTGTTGCTGCAAGTCAAGCTGCCTTAGGCTTATAACATCTACATTTAAAA
    1810      1820      1830      1840      1850      1860      1870      1880      1890      1900      1910      1920

GCATCTCAGCCTACCATGAGAATAAGAGAAAGAAAATGAAGATCAAAAGCTTATTCATCTGTTTTCTTTTTCGTTGGTGTAAAGCCAACACCCTGTCTAAAAAAACATAAAATTTCTTTAAT
    1930      1940      1950      1960      1970      1980      1990      2000      2010      2020      2030      2040

CATTTTGCCTCTTTTCTCTGTGCTTCAATTAATAAAAAAATGGAAAGAATCTAATAGAGTGGTACAGCACTGTTATTTTTCAAAGATGTGTTGCTATCCTGAAAATTCTGTAGGTTCTGTG
    2050      2060      2070      2080      2090      2100      2110      2120      2130      2140      2150      2160

GAAGTTCCAGTGTTCTCTCTTATTCCACTTCGGTAGAGGATTTCTAGTTTCTGTGGGCTAATTAAATAAATCACTAATACTCTTCTAAGTTAAAAAAAA
    2170      2180      2190      2200      2210      2220      2230      2240      2250      2        12        22
```

# Fig.4. 5' Non-coding region modification and signal peptide sequence excision

1. TaqI digest, fill-in, ligate BamHI linkers
   and isolate DNA fragment on a gel

2. PvuII/ClaI double digest, "fill-in"

\* Mature HSA

— cDNA

▢ pAT153 vector DNA

■ BamHI linkers

## *Fig.5.* Construction of an "authentic" HSA cDNA (MET-HSA)

M13 mp19 (Messing.J. 1983, Methods in Enzymology, 101, pp20)

PstI

5'————GTCGACTCTAGAGGATCCCCGGG——3'

3'————————————————————————5'
Hind III    Sal I    Xba I    BamHI    Sma I    EcoRI

SstI

XbaI digest

SI nuclease

Pst I   Sal I

   T 3'

   A 5'

Hind III

5' AGGATCCCCGGG————3'
3' TCCTAGGGGCCC————5'
  BamHI    Sma I    EcoRI

SstI

Hind III

5' GATC————ATCTCG 3'

3' ————————TAG AGCCTAG 5'
        ASP

HSA coding region

1843 bp BamH-I
fragment from
pAT I53ALB (Fig.3.)

Hind III    SI nuclease

5' ————ATCTCG 3'    Blunt end ligate

3' ————TAGAGC 5'

Pst I   Sal I   Hind III     junction

————ATCTCG|AGGATCCCCGGG————3'

————TAGAGC|TCCTAGGGGCCC————5'

Hind III   Junction

Sequencing
primer

5' CTCGAG   Unique XhoI site

XhoI digest

# Fig.5(Contd.)

0248637

```
5' ——————— ATC      TCGAGGATCCCCGGG ——————— 3'

3' ——————— TAGAGCT      CCTAGGGGCCC ——————— 5'
```

↓ S1 nuclease

```
5' ——————— ATC      GGATCCCCGGG ——————— 3'

3' ——————— TAG      CCTAGGGGCCC ——————— 5'
```

```
synthetic oligonucleotide   CATGGATCCATG
(12 mer)                     GTACCTAGGTAC
```

↓ insert, clone, sequence

```
Mp19.7met/9        12 mer        (Note: 5' TG 3' deletion see text)

5' ——————— ATC CAT GGATCCA GGATCCCCGGG ——————— 3'

3' ——————— TAG GTA CCTAGGT CCTAGG GGCCC ——————— 5'

           ASP MET  BamHI   BamHI
```

```
   ←————————————————————
   MET-HSA cDNA
```

↓ SalI digestion (3' site in the non-coding region of the M13mp19 cloning linker)

↓ DNA polymerase I (Klenow)

```
synthetic oligonucleotide   CATGGATCCATG
(12 mer)                     GTACCTAGGTAC
```

# Fig.5(Contd.)

insert, clone

M13mp19.7met/9.1

12 mer linker

5' — GTCGACATGGATCCATGTCGACTCAG ------------- ATCCATGGATCCAGGATCC —

BamHI    BamHI

3' — CAGCTGTACCTAGGTACAGCTGAGTC ------------- TAGGTACCTAGGTCCTAGG —

BamHI

ASP MET

MET-HSA cDNA

filled in SalI
site

0248637

# Fig 6  Plasmid pEK113

pEK113
6.2 kbp

B lactamase

P
E
H
B
H

MET-HSA

B

■■■ pBR322 DNA

☐ MET-HSA DNA

Restriction endonuclease sites
(see Fig. 1)

0248637

Fig.7 Construction of plasmid
pEHB11 MET-HSA

BamHI fragment
from pEK113

MET-HSA

BamHI

Ligase

# Fig.8 Construction of plasmid pET 13:1-MET-HSA

KEY: see figures 1 and 7

**Fig.9.** Galactose Induction of Gene Expression in NCYC240 (pEHBII)

β-Galactosidase Units

Time (Hours)

KEY

●—● Galactose+Glucose

○--○ SDCO9(1:10)

X—X Galactose

# Fig.10. Plasmid pEK1014 ALB1

E. coli plasmid pAT153 DNA, ■■■ ; yeast chromosomal DNA,
▭ ; N-methionyl human serum albumin coding sequence from
pEK113, ▭ ; GAL10/CYC1 hybrid promoter, ▨ ; CYC 3'
transcription terminator, ▭ ; yeast 2μm plasmid DNA, ——;

GAL10/CYC1 — N-methionyl human serum albumin fusion: DNA sequence

```
    5'                               Met Asp    3'
  - TTAATAATAACTGAATTCCCGGGGGATCC ATG GAT _ _ _ _ _
        CYC1 5'                      N-Met-HSA
```

# Fig.5(Contd.)

```
5' ——————— ATC        TCGAGGATCCCCGGG ————————— 3'

3' ——————— TAGAGCT        CCTAGGGGCCC ————————— 5'
```

↓ S1 nuclease

```
5' ——————— ATC          GGATCCCCGGG ————————— 3'

3' ——————— TAG          CCTAGGGGCCC ————————— 5'
```

synthetic oligonucleotide    CATGGATCCATG
(12 mer)                     GTACCTAGGTAC

insert, clone, sequence

↓

Mpl9.7met/9        12 mer        (Note: 5' TG 3' deletion see text)

```
5' ——————— ATC CAT GGATCCA GGATCCCCGGG —————— 3'

3' ——————— TAG GTA CCTAGGT CCTAGG GGCCC —————— 5'
```

ASP MET   BamHI   BamHI

← MET-HSA cDNA

↓ SalI digestion (3' site in the non-coding
   region of the M13mp19 cloning linker)

↓ DNA polymerase I (Klenow)

synthetic oligonucleotide    CATGGATCCATG
(12 mer)                     GTACCTAGGTAC